# EUROPEAN PATENT SPECIFICATION

(11) **EP 4 162 967 B1**
(45) Date of publication and mention of the grant of the patent: **15.07.2026**
(21) Application number: 21201731.3
(22) Date of filing: 08.10.2021
(51) Int. Cl.: A61M 25/10, A61M 25/00, A61B 17/22

(54) **BALLOON CATHETER SYSTEM**
BALLONKATHETERSYSTEM
SYSTÈME DE CATHÉTER À BALLONNET

(43) Date of publication of application: 12.04.2023
(73) Proprietor: WeMed Technologies GmbH, 40210 Düsseldorf (DE)
(72) Inventor: ALNIDELIK, Ahmet, 40625 Düsseldorf (DE)
(74) Representative: Tergau & Walkenhorst Intellectual Property GmbH

(56) References cited:
- EP-A1- 0 025 349
- EP-A1- 2 425 870
- WO-A1-2008/123937
- WO-A1-2014/022867
- WO-A1-2019/027380
- WO-A1-2019/165214
- WO-A1-99/44686
- WO-A2-2007/090863
- US-A1- 2004 230 316
- US-A1- 2011 218 494
- US-A1- 2015 306 344
- US-A1- 2017 281 140

## Description

The invention relates to a balloon catheter system, in particular a thrombus catheter, preferably a recirculating transferring dilatation catheter system, used in the treatment and cleaning of arteries, veins, other circulatory channels in the circulatory system of humans and animals, digestion, dilatation, fluid delivery, medication delivery, plaque, stenosis, thrombus and other extra anatomical, extra biological structures and formations.

Catheter devices of this type, and more particularly catheter devices comprising multiple inflatable means carried by the catheters, are widely used in treatment concepts or methods for the site-specific local delivery of agents to biological spaces in medical procedures. In particular, such catheter systems, preferably with at least one radial opening located between at least a pair of inflatable means, may be used in methods for site-specific or local delivery of agents into blood vessels (including the blood vessel lumen and the vessel wall) for treatment of said blood vessels and/or other organ systems, as well as methods of visualizing the lumen of said blood vessels and/or other organ systems.

Arterial obstruction and constriction, venous obstruction, stenosis and venous insufficiency, narrowing in the excretory system, are defined as complaints caused by extra anatomical and extra biological conditions. Arterial stenosis or occlusion is an artery disease. These diseases occur throughout the neck (carotid), arm (subclavien, axillary, brachial, radial), abdominal vessels (Abdominal aorta, common iliac, external iliac, internal iliac) and leg vessels (femoral artery, Sfa, profunda or DFA, popliteal artery, anterior tibial artery, peroneal artery, posterior tibial artery). Since the blood flow is slowed or completely interrupted beyond the area where the congestion or narrowing occurs in these diseases that occur in the arteries, it can not be fed sufficiently after the occlusion, causing resting pain, severe pain while walking, discoloration of the skin, and finally loss of limb. Venous occlusion, thrombus or insufficiency is a venous disease. The most important venous occlusion, thrombus and insufficiency occur in the veins in the leg and abdomen. In addition, venous occlusion and thrombus are also seen in the vein vessels of the arm. In case of occlusion, thrombus or insufficiency, there is pain, swelling and discoloration of the skin in the relevant area, but it can result in severe paralysis or death by causing stroke or pulmonary obstruction in the brain due to clotting in the future.

Typical treatments performed nowadays for artery and vein vascular occlusions, thrombus formation or narrowing are: By-Pass surgery is in the form of PTA balloon dilation, atherectomy, aspiration, bare or covered stenting and/or dilatation with medication loaded balloons in endovascular treatments, allowing the coated medication to release in the lesioned area.

A very common treatment known for the aforementioned diseases is medication transfer to the diseased area with a medication-coated balloon. The patient is placed on the imaging device (angio and fluoroscopy) table in the imaging laboratory. Necessary antisepsis is applied to the relevant area to be introduced. With the Seldinger technique, intravascular cannulation is provided by introducing into the vessel, in particular arteri or vein, and placing the introducer sheath. All subsequent operations to be performed and all devices to be used are sent through this sheath. A contrast agent that helps diagnostic imaging is injected into the vessel through the sheath or with the help of a catheter, and the vein image is provided under fluoroscopy. In this way, the occlusion, stenosis, thrombus, plaques, calcification and/or the diseased area in the vessel are diagnosed and determined. This area is passaged with the help of guidewires and/or catheters and intravascular full cannulation is provided. With the guidewire in the vessel, a PTA balloon catheter is sent over it, inflating with ATM (atmospheric) pressure suitable for the effect of the balloon for pre-dilatation, the relevant area is expanded and the balloon is deflated after waiting for the desired time. The same process is repeated if this expansion is not sufficient. When the opening of the relevant area in the vessel is deemed sufficient, a balloon catheter loaded with paclitaxel, sirolimus,everolimusor similar medication is sent and the diseased area is inflated for the time specified in the technical table, allowing the medication coated on the balloon to pass to the vessel surface.

This medication delivery process aims at minimizing re-occlusion condition. In case the diseased area within the vessel cannot be reached the desired width despite all these procedures, With the help of various atherectomy devices, the plaque load in the vessel is tried to be cleared, or the optimum width within the vessel is tried to be achieved by placing a loaded stent or a self-expandable stent on the balloon in order to provide the desired diameter without atherectomy.

Some main issues in these concepts may occur during the medication-coated balloon application, which is among these treatment steps. The medication loaded on the balloon with the help of various chemicals is subjected to various processes while being sent into the vessel. While the balloon is removed from the sterile package and loaded to the guide wire, hand and fluid contact occurs on the balloon surface, while it is sent to the target area over the guidewire, it passes through a tight introducer sheath filled with blood or fluid, and a certain surface of the balloon is completely in contact and interacts with blood during the time it goes to the target area. In all these steps, the medication covered on the balloon suffers serious losses due to these interactions and cannot be transferred to the vascular surface in targeted doses and amounts. Medication of the part of the balloon surface may completely disappear. As the current technology is insufficient some medication types with certain characteristics can not be used to cover balloons.

There are some catheters created as a solution to the problems mentioned above. The general purpose of the catheters in question is to compress the problematic area inside the vessel with balloons inflated from both ends, to clean the area with the aspiration process, and then to transfer the medication administered through the lumens to the relevant area.

European patent application No. EP2 497 524 relates to a multi-balloon catheter for medication delivery into veins. It disclsoes a multi-balloon catheter for delivering a therapeutic and/or diagnostic agent to tissue, comprising a first balloon, a second balloon, and a catheter having a third balloon positioned between said first and second balloon. It has a fluid supply thereto to create a chamber on the catheter shaft between said first and second balloon, with fluid providing to inflate the first and second balloons mentioned. The catheter mentioned has a fluid path between the first balloon and the second balloon through which it can deliver the therapeutic and/or diagnostic agent, and a fluid source that increases the fluid pressure into the chamber by providing fluid to the said third balloon, and aims to transfer the administered medication into the tissue by inflating the middle balloon.

US 6,485,500 discloses an embolism protection system designed to isolate a part of a blood vessel and prevent embolism from migrating into the rest of the vascular system. It comprises a distal blocking balloon catheter with pores at the proximal end of the catheter and an inflation feed fluid in fluid connection with distal and proximal barrier inflatable balloons. Proximal to the blocking balloon the catheter is adapted to be positioned in the vascular system and to provide inflation flow by rapidly guiding the inflation fluid under pressure from said pores and emboli to the emboli in the isolated part of the blood vessels towards the exit ports of the proximal blocking balloon catheter and close to the fluid communication lumen of the proximal blocking balloon catheter.

WO 2010/062778 A2 discloses a catheter comprising multiple inflatable balloons, in which the center balloon positioned between a proximal and a distal balloon is provided in order to minimize the surrounding volume within the blood vessel. When the medication is delivered via skyve ports positioned on the catheter shaft between the proximal and distal balloon, the center balloon may be inflated to minimize said volume, thereby improving the efficiency of medication delivery.

In WO 2019/027380 A1, a catheter system comprising multiple inflatable balloons is disclosed in which the center balloon is provided with a porous skin, thereby enabling the use of the center balloon as delivery port for the agent or medication.

In all these systems, the catheter system comprises a catheter shaft with a number of lumens each of which provides a media channel to either one of the balloons or to orifices positioned on the catheter shaft in the medication delivery segment. Accordingly, by means of the mutli-lumen catheter shaft, dilatation, aspiration, fluid, and/or fluid-medication may be delivered to the respective areas on the catheter shaft for their respectvive purposes.

It is now an objective of the present invention to provide a catheter system of the type identified above having a particularly simple, easily usable, cost efficient design allowing for low production cost while providing high reliability nevertheless. In particular, the invention aims at having a particularly effective use during the treatment. the patient should be given the best, safest and fastest treatment while at the same time the system should be user friendly for the doctors. The goal is to first of all offer a catheter system with multiple, preferably unlimited, options of medications compared to a now limited list of options of medication.

In accordance with the invention, this objective is achieved by providing a catheter shaft for the catheter system according to claim 1, said catheter shaft being assembled from a number of tubes which are bundled together by adhesive force to form the shaft body. In the concept of the present invention, bundling or bonding by "adhesive force" is supposed to mean any technology of substance-to substance bonding of components, such as by adhesive, glue, soldering, welding, thincoating, vulcanizing or the like. Preferably, bonding is effected by medical grade adhesive. Preferred embodiments are defined in the dependent claims.

Preferred embodiments of the invention are specified in the dependent claims. The invention is based upon the consideration that minimization of production costs may be achieved by keeping as many components particularly simple as possible. Under this aspect, in the catheter systems as known from the prior art, the multi-lumen catheter shaft is recognized as a relatively complex, costly component that under the consideration of the present invention should be simplified. In particular, for the multi-lumen catheter shaft, independence and reliable separation of each lumen against each other is important for proper functionality of the system since the respective media transported via the lumen must be kept separate from each other in order to enable reliable and efficient treatment.

In the existing systems, the catheter shaft is manufactured by extrusion. This, however, requires complex treatment since a number of up to five lumens must be integrally formed into the extruded shaft body. In order to simplify production and minimize cost, it is therefore an aspect of the present invention to use a number of originally separate tubes to provide for the lumens, and assemble them together into the actual catheter shaft by adhesive means, in particular by welding or glueing, or any other appropriate technique. It can be combined with coating or an extra external tubing. Welding may vary by bounding.

According to the invention, three tubes are assembled to form the catheter shaft. In order to achieve a particularly compact desing, the individual tubes are arranged such that, as seen in cross section, their respective centers are the corners of an equilateral triangle. Further according to the invention, the tubes forming the catheter shaft are surrounded by an outer coating, encapsulating the set of assembled tubes.

Preferably, one, some, or all of the tubes each providing a single lumen comprises a multi-layered tube wall. In this preferred embodiment, the wall of the respective tube is a multi-layer structure, in which several layers with different functionality are provided. Preferably, at least one of said layers, e. g. a middle layer, is made from reinforced, e. g. braided, material such as braided PTFE, HDPE, or PFA, thereby providing enhanced mechanical strength, durability, and resistance. In contrast, another of said layers, e. g. an inner layer facing towards the lumen and thereby potentially contacting the medium to be transferred, may be made of material such as PTFE, HDPE, or PFA with low frictional resistance. Yet another layer, e. g. an outer layer surrounding the other layers, may be made of materials such as PTFE or PET with low frictional resistance and adding rigidity to the tube system.

A preferred triple tube design can be made with two different tube sizes, this not falling under the invention as claimed, two occlusion balloons with the same characteristics, a perforated middle balloon and standard connectors available in the market, in particular standard or specially designed handheld Y connectors.

In another aspect of the disclosure, the central balloon of the catheter system is designed as a porous balloon that may be used as a delivery port for the agent or medication into the blood system included between the distal and proximal occluding balloons. In accordance with this aspect of the invention, the outer wall of the central balloon may be equipped with holes in various sequencing designs and have different hole sizes to transfer any drug and/or liquid to the diseased area in the vessel. For example, the arrangement of the holes can be straight, spiral, complex or in any other geometric shape. The diameter of the holes in a preferred embodiment may range from 1 micron to 50 microns. The perforated balloon in the middle in a preferred embodiment can be produced smaller than the diameter of the treated vessel or suitable for the vessel diameter. This production logic will ensure that the drug and/or liquid to be sent into the vein will be applied 360 degrees on the inner surface of the vein in the most effective way. The fact that the perforated balloon in the middle is smaller than the vessel diameter will prevent a barotrauma, to which the vessel is exposed. Because, in a kind of dilatation procedure in the vein creates a trauma in the vein and the vein gives a vasospasm reaction to it. This reaction can cause acute occlusion even if a successful operation is performed on an already occluded or diseased vessel.

In yet another independent aspect of the disclosure, the catheter system is equipped with a circulation system for the agent. This re-circulation system in principle works according to the following steps (method not claimed):
1. The balloon catheter shaft is sent to the target area detected in the vessel.
2. In order to inflate the distal and proximal occlusion balloons, an inflator or similar device is connected to a first extension tube connected to the hub.
3. Distal and proximal occlusion balloons are inflated, resulting in isolation of the area of the perforated balloon in the middle from the rest of the blood system.
4. Remaining blood in the isolated area can be aspirated via the aspiration lumen.
5. The inflator device is connected to the respective tube in order to inflate the perforated balloon in the middle and/or transfer the drug and/or liquid to the target area.
6. The perforated balloon in the middle is inflated and/or the drug and/or fluid is being transferred. The drug and/or liquid delivered with a controlled pressure begins to further inflate the perforated balloon. When the pressure applied reaches a certain level, the drug and/or liquid starts to fill the isolated area formed by the distal and proximal occlusion balloons inflation through the holes of the perforated balloon. When the filling reaches the appropriate pressure within the second extension tube to which is connected, a retrograde flow occurs within the aspiration channel in between the distal - and perforated balloon in the middle. The flow is maintained until it exits the proximal of the second extension tube. Afterwards, the second extension tube is inserted into a Y-connector connected to the third extension tube with a one-way valve that allows a retrograde flow. After this connection, the inflator device connected to the third extension line ensures the recirculation thanks to the pressure created in a controlled manner.

As another option, the second extension tube may be connected to a circular motor pump. The tube is connected to the rotating part of the motor pump which provides re-circulation by the rotation which is provided, similar to a dialysis device.

According to the appropriate method (not claimed) in dialysis treatment, the second extension tube is connected to the circular motor section. The appropriate engine rotation speed is determined in the tests. According to the determined rotation speed, the motor pump provides effective recirculation of the drug and/or liquid in the localized area in the vessel.

The main advantages achieved with the invention are that the multi-lumen tube design is very simply and economic, and it has great advantages in terms of shaft, production, production expenses (machine and equipment, components, number of employees), R & D expenses (prototyping, preliminary functional tests, clinical studies). The shaft can be formed by placing several tubes of same diameter and same properties on top of each other by applying medical grade adhesive and thin heat shrink coating on it. Various individual tubes can be the same original tube cut in different lengths to form a catheter shaft. According to the performance evaluation, these tubes can be selected from materials with different parameters with different wall thicknesses or reinforced to increase push ability and kink resistance. Desired features can be added to single lumen tubing. As a major advantage, operation cost and time will be reduced.

If necessary, balloon deflating and inflation times can be adjusted or improved by adjusting free cross section of the tubing in the occlusion balloon tube line. During the production stages, wires (mandrel) may be put into the pipes, providing comfortable working opportunity. During all production processes tubings can stand rigid and lumen collapsation does not occur, whereas pushability and kink resistance are increased.

In terms of producing the product, it provides great convenience compared to conventional multi-lumen shaft designs based upon extrusion processes. Very precise holes or channels must be drilled on the multi-lumen shaft for inflating and deflating the occlusion balloons and the perforated balloon, drug delivery and aspiration. In the multi-tube design according to the present disclosure there will be no need to drill holes on the shaft; it is only necessary to perforate a hole for the first occlusion balloon. In the triple pipe design, the liquids are sent independently of each other in a cylindrical geometry, which is efficient and more advantageous in terms of inflation and deflation times. The inner and outer surfaces of the independent pipes in a preferred embodiment can be coated with materials with low friction resistance.

In particular, the design of the catheter shaft according to the invention is less costly and allows for more effective operations as compared to previously known systems. In aprticluar, the new catheter design, created by combining separate tubings according to the multilumen catheter shaft, provides wider interior lumens and shortens the inflation and deflation times of balloons, thereby increasing pushability. Thanks to these larger interior lumens the drugs and/or liquids are delivered to the diseased area in the most effective way and recirculation is carried out.

Another important feature of the subject matter of the disclosure is that it can be re-used in the same patient. In peripheral artery or vein diseases, the lesions may be very long and bilateral. In this case, it may be necessary to use more of the drug-coated balloons. This increases the cost of the procedure and also requires a continuous catheter exchange in the diseased vessel.

During these changes, the guidewire, guiding sheat, and/or balloon catheter can come into contact with the vessel surface. This contact may cause vaso spasm or a different complication that may occur in the vessel. This disclosure enables treatment with minimal catheter changes in the same vessel. Thanks to its perforated balloon design, the catheter shaft enables effective drug transferring with less lumen in within a smaller tube, applying unlimited drug options and recirculation with addition of any technology provided in the future.

Exemplary embodiments are explained in further detail by reference to the drawings. In the drawings, it is shown in:
- Fig. 1: a catheter system in perspective view,
- Fig. 2: the catheter system of Fig. 1 in top view,
- Fig. 3: the catheter system of Fig. 1 as seen in direction of tip,
- Fig. 4: the catheter system of Fig. 1 as seen from behind,
- Fig. 5: the catheter system of Fig. 1 in longitudinal section,
- Fig. 6: an expanded excerpt of Fig. 5,
- Fig. 7: a blood vessel segment with lesion in longitudinal section,
- Fig. 8: a balloon segment of a conventional PTA catheter system before insertion into the vessel segment of Fig. 7,
- Fig. 9: the vessel segment of Fig. 7 with the balloon segment of Fig. 8 inserted,
- Fig. 10: the vessel segment of Fig. 7 with the balloon segment inflated,
- Fig. 11: the balloon segment of the conventional PTA catheter system after removal from the vessel segment of Fig. 7,
- Fig. 12: the blood vessel segment of Fig. 7 after treatment with the conventional PTA catheter system with the remaining lesion and with the balloon segment of the catheter system of Fig. 1 before insertion in longitudinal section,
- Fig. 13: the catheter system of Fig. 1 in the phase of creating a closed area in a diseased region by inflation of occlusion balloons,
- Fig. 14: the catheter system of Fig. 1 in the phase of aspiration the blood remaining in the closed area created in the phase shown in Fig. 13,
- Fig. 15: the catheter system of Fig. 1 in the phase of delivering medication to the closed area in a diseased between the occlusion balloons via the treatment perforated balloon after the aspiration phase shown in Fig. 14,
- Fig. 16: the vessel of Fig. 12 with introduced treatment balloon in longitudinal section,
- Fig. 17: the vessel of Fig. 12 with introduced treatment balloon in cross section,
- Fig. 18: the catheter system of Fig. 1 in the phase of drug circulation and aspiration stage,
- Fig. 19: the catheter system of Fig. 1 with deflated occlusion and center balloons, ready for removal from the blood vessel,
- Fig. 20a-20c: the shaft of the catheter system of Fig. 1 in cross section, of which Fig. 20b discloses a catheter system according to the invention,
- Fig. 21a-21b 22: the catheter shaft of the catheter system of Fig. 1 being passed through an introducer sheath, in cross section,
- Fig. 23a-23b: an individual tube of the catheter shaft of the catheter system of Fig. 1 in cross section,
- Fig. 24: a catheter system in re-circulation mode.

Identical parts are identified by identical reference numerals in all Figs.

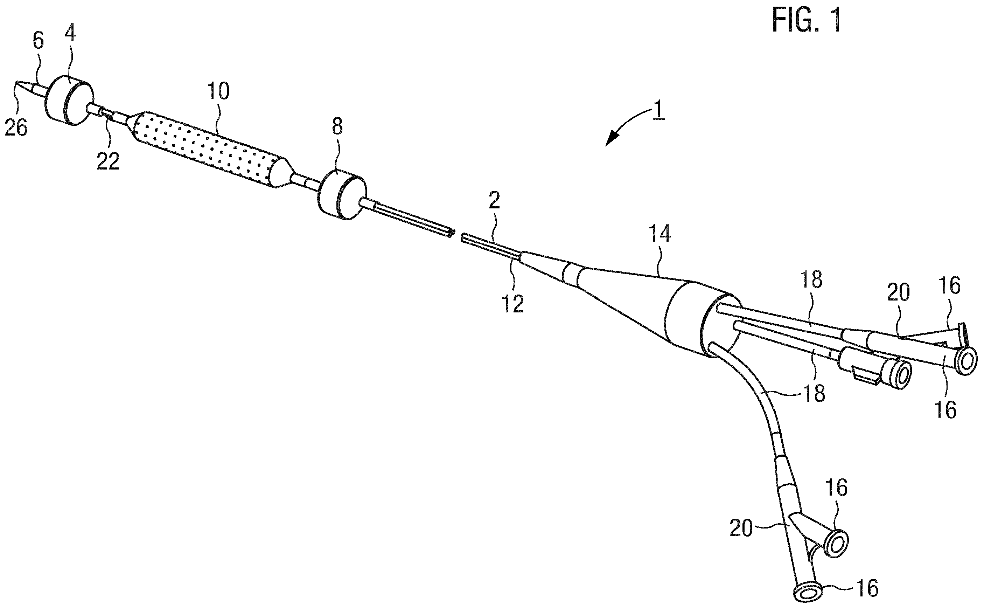

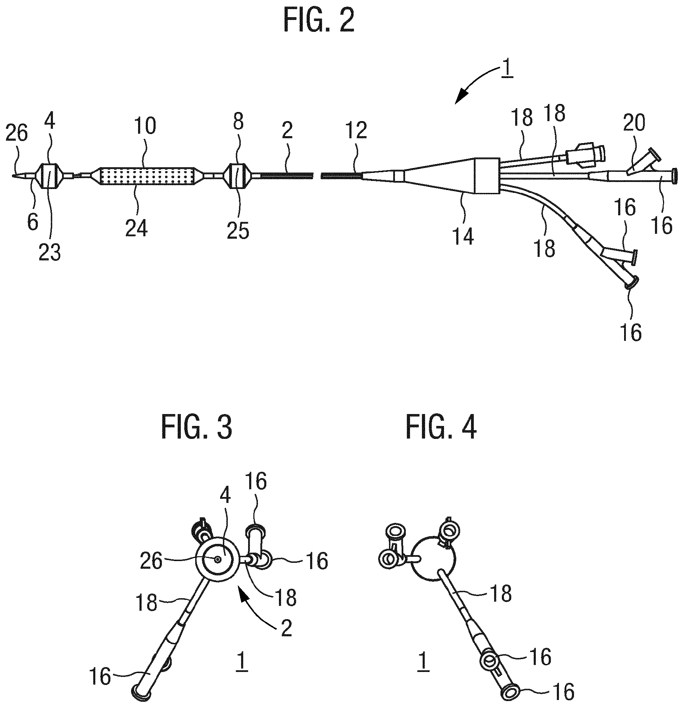

The catheter system 1 as shown in Figs. 1 - 5 is designed for use in treatment methods or procedures for treatment of diseases caused by lesions, occlusion, constriction, thrombus, plaque, stenosis, extra anatomical and extra biological conditions in arteries and veins. In the treatment method enabled by the catheter system 1, hydrodynamic principles to deliver fluid, medication, pressure for dilatation, aspiration and circulation to the diseased area caused by lesions, occlusion, stenosis, thrombus, plaque, extra anatomical and extra biological conditions in the arteries, veins, or other circulation channels may be used.

The catheter system 1 comprises multiple inflatable means located on a common catheter shaft 2: a distal inflatable balloon 4 located in the vicinity of the distal end 6 of the catheter shaft 2, a proximal inflatable balloon 8 located on the catheter shaft 2 at a disctance from the distal balloon 4, and a center inflatable balloon 10 located on the catheter shaft 2 between the distal balloon 4 and the proximal balloon 8. The distal balloon 4 and the proximal balloon 8 are designed as occluding balloons; when inflated, they will expand until they contact the inner surface of the respective blood vessel and seal it off, thereby providing a sealed, isolated volume in the blood vessel in the segment between them. The center balloon 10, in contrast, is designed as treatment balloon.

The catheter shaft 2 of the catheter system 1 is designed as a multi-lumen shaft 2 and at its proximal end 12 is connected to a multi-lumen hub 14 that on its inlet side is connected to a number of connectors 16, in the embodiment shown female Luer connectors 16, via a number of individual the tubings 18. Depending on the individual design and layout of the end-part of the catheter system 1, the tubings 18 may also be interconnected by a number of Y-connectors 20 or branch-offs, as shown in Figs. 1 - 5. In its distal region, in the section between the distal balloon 4 and the center balloon 10, the catheter shaft 2 is provided with an orifice or aspiration pore 22 for dispensing an agent or medication. Further, the catheter shaft 2 has a number of marker bands 23, 24, 25 to mark individual positions on the catheter 1, e. g. the positions of the balloons 4, 8, and a soft or atraumatic tapered tip 26 at its distal end 6. In the embodiment shown, in an aspect considered independently inventive, three (or more) marker bands 23, 24, 25 are provided, wherein first marker band 23 is associated with distal balloon 4, second marker band 24 is associated with center balloon 10, and third marker band 25 is associated with proximal balloon 8. In this inventive embodiment, the combination of marker bands 23, 24, 25 and their respective positioning on catheter shaft 2 allow for improved, enhanced monitoring and, consequently, enhanced treatment precision.

In the embodiment as shown, the distal balloon 4 and the proximal balloon 8 are pre-welded on the catheter shaft 2, enabling them to cover both ends of a lesion or target area.

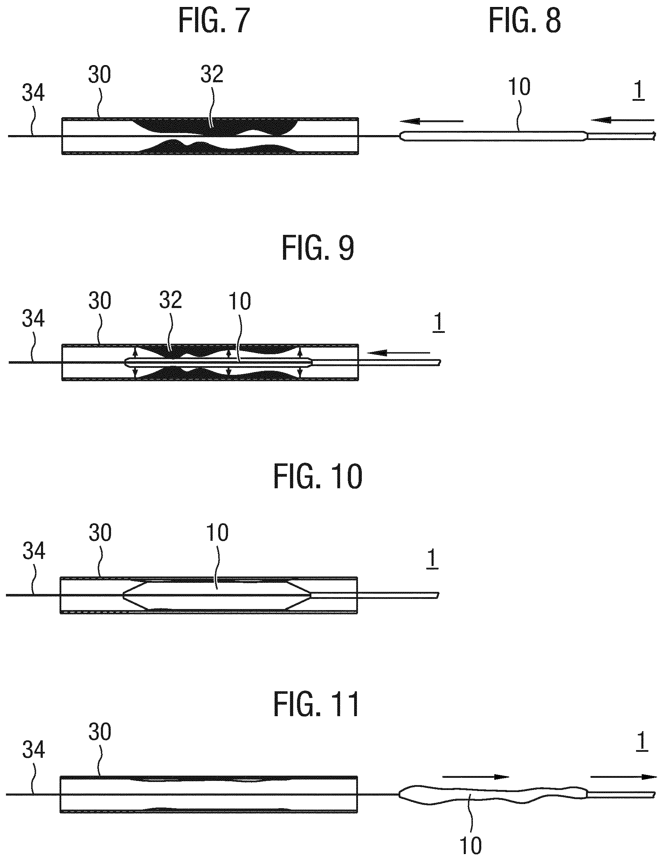

Figs. 7 to 11 show a sequence of the main steps of a pre-treatment concept using a conventional PTA balloon catheter system intended to widening the lesioned section on a mechanical basis. In particular, Fig. 7 shows a segment of a blood vessel 30 with a lesion 32. Correspondingly, in Fig. 8 the end segment of (conventional) PTA catheter system is shown ready to be inserted into the vessel segment. Once the end segment of the PTA catheter has been introduced onto a guidewire 34 (used to assist in guiding the catheter to the target segment of the blood vessel 30) and sent to the diseased area through an appropriate introducer sheath placed percutaneously or through the surgically explored vessel, the balloon of the PTA catheter system is positioned in the diseased area with the lesion 32 and is then inflated via a corresponding lumen in its associated catheter shaft 2, as shown in Fig. 9. The PTA balloon then for treatment purposes may be inflated to full size, as shown in Fig. 10. After treatment, the treatment balloon may be deflated again, and the PTA catheter is removed from the blood vessel 30 (Fig. 11). Inflating of the balloon in this procedure may be effected by appropriate general means such as pump, injector, and in-/deflator devices.

In this pre-treatment procedure, the PTA balloon or treatment balloon may, as shown, be used as a mechanical device; in this case inflation of the treatment balloon will exert mechanical force on the lesion 32, thereby potentially shrinking it. In the subsequent treatment phase with the catheter system 1 of the present invention, the balloon segment comprising balloons 4, 8, and 10 may be introduced into the lesioned vessel segment. In this treatment procedure, the center balloon 10 or treatment balloon may, as shown, be used as a mechanical device; in this case inflation of the treatment balloon, in addition to potentially exerting mechanical force on the lesion 32, will reduce the free volume within the encapsulated volume between the occluding balloons 4, 8. In this status, aspiration and /or the agent or medication may be delivered to the encapsulated, minimized volume via the orifice 22; due to minimization of the free volume in this area, distribution of the medication in the blood and/or adherence of the drug on the vessel surface will be much more efficient, which may be highly beneficial in case of delicate or expensive medication.

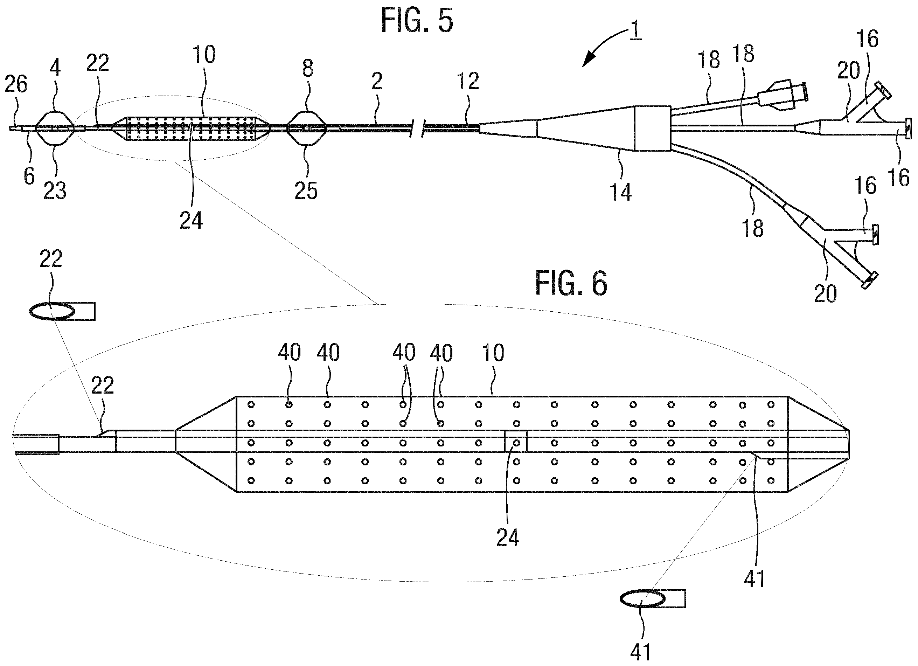

In one inventive aspect of the present disclosure, however, the treatment balloon 10 as such is designed as delivering means for delivering agent, medication, or other media directly into the volume within the blood vessel 30. For this purpose, as can be seen in Fig. 5 or in the expanded view in Fig. 6, the balloon 10 is designed as a porous balloon, i. e. its outer wall is provided with pores 40 and/or made of porous material. This design, in particular with even or homogenous distribution of the pores 40 or holes across the entire surface of the balloon 10, will ensure that the drug or liquid to be sent into the vein is applied 360 degrees to the inner surface of the vein in the most effective way. Fig. 6 also shows the inflation/deflation tube 41 for the center balloon 10.

In a preferred embodiment shown, the perforated balloon 10 in the middle is smaller than the vessel diameter in order to minimize the risk of barotrauma to which the vessel is exposed. In particular, any kind of dilatation procedure performed in the vein creates a trauma in the vein and the vein gives a vasospasm reaction to it. This reaction can cause acute occlusion even if a successful operation is performed on an already occluded or diseased vessel. Another important feature of the invention is that it can be reused in the same patient. In peripheral artery or vein diseases, the lesions may be very long and bilateral. In this case, it may be necessary to use more of the drug-coated balloons. This increases the cost of the procedure and also requires a continuous catheter change in the diseased vessel. During these changes, Guide wire, guiding sheat, balloon catheter come into contact with the vessel surface. This contact may cause vaso spasm or a complication that may occur in the vessel. The present invention enables treatment with minimal catheter exchange in the same vessel. Thanks to its perforated balloon design, the catheter shaft enables effective drug transfer with less lumen in a smaller tube, applying unlimited drug options and recirculation with the possibilities provided by the technology in the future.

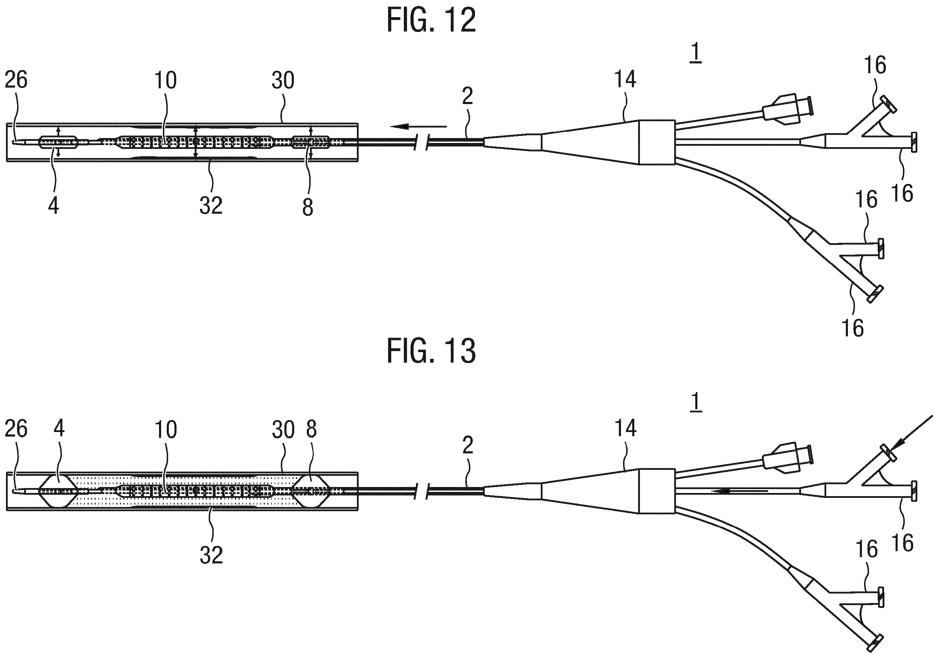

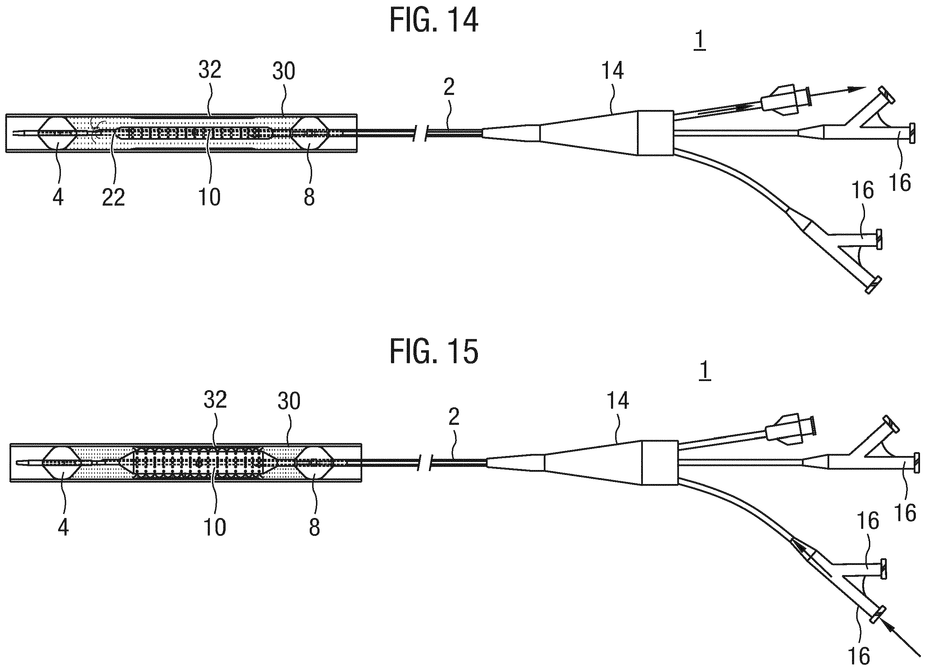

Figs. 12 to 20 show a second sequence of the main steps of a treatment concept using the catheter system 1 with the center balloon 10 desigend as a porous treatment balloon 10. Fig. 12 shows the pre-treated segment of the blood vessel 30 with the lesion 32 in which the end segment of the catheter system 1 is being inserted into the vessel segment. Once the end segment of the catheter 1 has been sent to the diseased area through an appropriate introducer sheath placed percutaneously or through the surgically explored vessel, the distal occlusion balloon 4 and the proximal occlusion balloon 8 are positioned in the diseased area with the lesion 32 such that they can encapsulate the lesion 32. They are then inflated via the lumen in the catheter shaft 2, thereby sealing off the blood vessel 30 on both sides of the lesion 32, as shown in Fig. 13. Then, in a stage of aspiration shown in Fig. 14, the blood remaining in the closed off segment between the occlusion balloons 4, 8 may be aspirated via the aspiration pore 22. The center balloon 10 then for treatment purposes may be inflated as well, as shown in Fig. 15.

In other words, for the treatment as shown, the distal and proximal balloons 4, 8 are inflated to close off the area to be treated such that fluid, fluid-medication, agent, or the like, may be delivered, in particular by applying pressure using hydrodynamic principles, aspiration, circulation. The fluid or blood remaining in the area of the aspiration pores 22 may be simultaneously aspirated in the area between the two balloons 4, 8 (phase shown in Fig. 14). Accordingly, the aspiration pore 22 is configured to aspirate the interballoonal fluid, blood in the segment between the two balloons 4, 8. Then, in the phase shown in Fig. 15, the fluid, fluid-medication mixture also may be injected through the porous balloon 10 via respective tubing using any one or more of pump, injector and inductor devices.

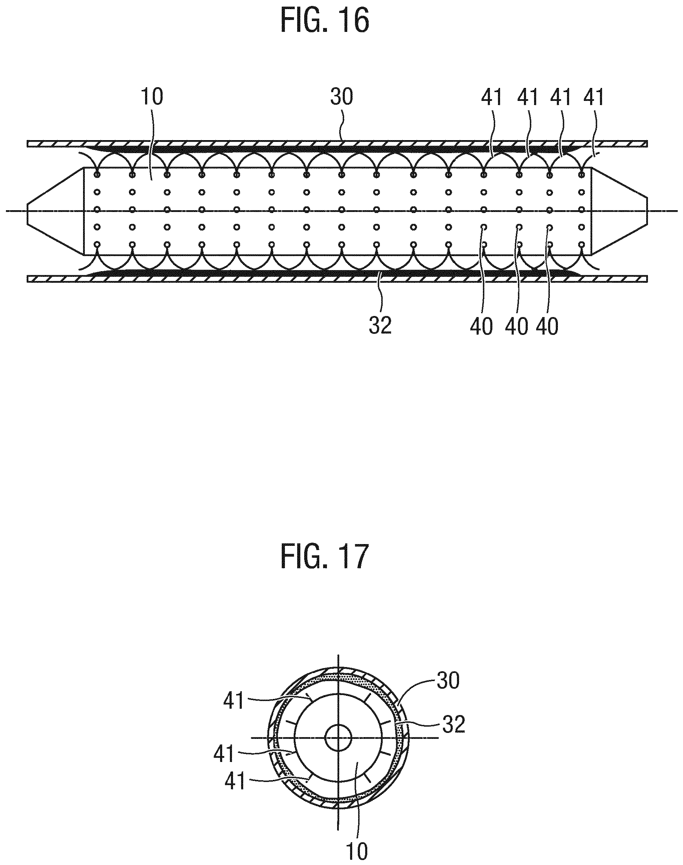

The porous balloon 10 in this phase is shown in more detail in longitudinal section in Fig. 16 and in cross section in Fig. 17. The drug delivered to the free volume surrounding inflated balloon 10 within the vessel 30, as indicated by arrows 41, is passed through holes or pores 40 and thereby can be delivered directly to the region of the lesion 32.

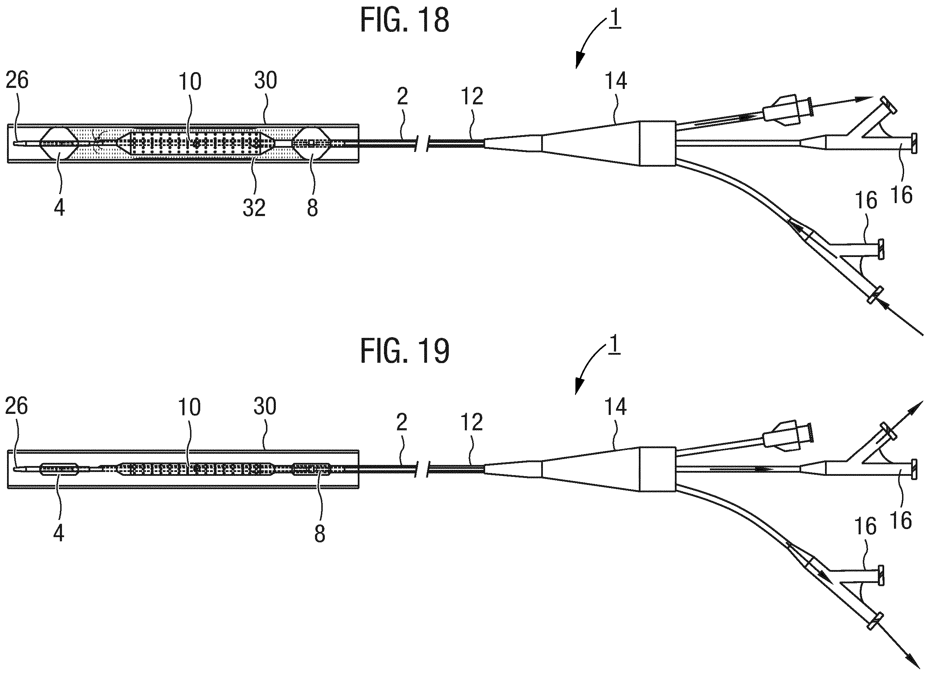

After delivery of the drug or medication, in a subsequent treatment phase shown in Fig. 18, the drug or medication together with aspiration may be circulated. Thus, the process may also provide circulation of the fluid, fluid-medication mixture in the segment between the distal balloon 4 and the proximal balloon 8 and the application of dilatation with hydrodynamic pressure to the vascular surface in the diseased area by virtue of the controlled circulation. By virtue of the pressure to be applied to the vessel surface using hydrodynamic principles, it ensures that dissection and plaque flaps that may occur during pre-dilatation or post-dilatation procedures adhere more effectively to the vessel surface. Thanks to hydrodynamic pressure, in the treatment of intraluminal dissections and flaps, the need for long-term pta balloon application and/or stent placement, which the vascular structure perceives as unfamiliar and reacts, is eliminated or at least reduced.

After treatment, all balloons 4, 8, 10 may be deflated again, and the catheter 1 is removed from the blood vessel 30 (Fig. 19). Inflating/deflating of the balloons 4, 8, 10 in this procedure may be effected by appropriate general means such as pump, injector, and in-/deflator devices.

In order to safely and reliably provide the functions described above, proper and controlled supply and delivery of various media to the respective parts is necessary and important: fluid and/or medication must be delivered to the balloons 4, 8, 10 and to the aspiration pore 22. In order to provide for this, in one aspect of the present invention the catheter shaft 2 is designed as a multi-lumen shaft 2 in the way of a multi-pipe or multi-tube design, comprising an occlusion balloons lumen 42, intended to deliver medium to the occlusion balloons 4, 8, a treatment balloon lumen 44, intended to deliver medium to the center balloon 10, an aspiration lumen 46, intended to deliver medium to the aspiration pore 22, and a guidewire lumen 48 for the guidewire 34. At the proximal end 12 of the catheter shaft 2, they are all connected to the multi-lumen hub 14, which in turn is connected to the respective tubings 18 with the connectors 16 for attachment to appropriate medium reservoirs or delivery devices such as pumps etc. At their distals ends, each lumen 42, 44, 46, 48 is connected to the respective balloon 4, 8, 10 or aspiration pore 22, respectively, thereby forming a treatment balloon tubing line, a guidewire tubing line, a distal and proximal balloon inflation, inflation & deflation tubing line, and an aspiration tubing line, all of which are integrated into the multi-lumen catheter shaft 2. Consequently, in the embodiment shown the multi-lumen catheter shaft 2, which is configured with a valve distal to the guidewire line, is designed as a four-lumen catheter shaft 2, which is considered to be highly efficient and therefore preferred.

In one inventive aspect of the present invention, the catheter system 1 features a particularly simple, cost efficient design allowing for low production cost while providing high reliability for medium supply to the treatment components nevertheless. In order to achieve this objective the catheter shaft 2 is assembled from a number of individual tubes 50 which are bundled together by medical grade adhesive to form the body of catheter shaft 2.

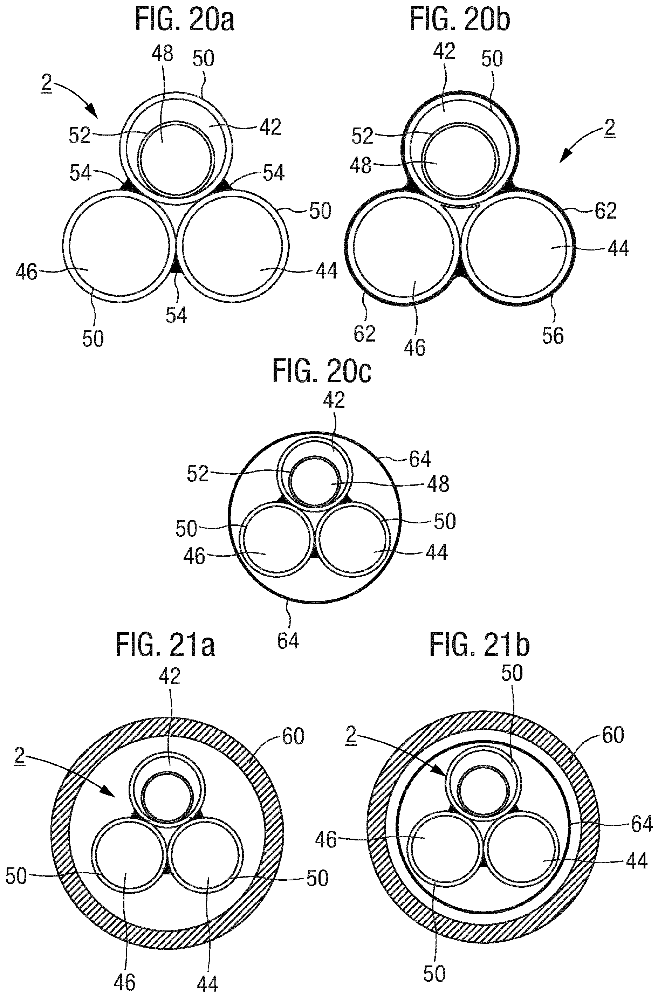

This particular design of the catheter shaft 2 becomes evident from the cross sectional view in Fig. 20. In the embodiment shown in Fig. 20a, the shaft 2 is formed by three tubes 50, one of which defines the occlusion balloons lumen 42, the treatment balloon lumen 44, and the aspiration lumen 46, respectively. The guidewire lumen 48 is defined by a smaller-size tube 52 positioned inide the tube 50 forming the occlusion balloons lumen 42. In the particular embodiment of the disclosure shown in Fig. 20, a particularly compact design for the shaft 2 is achieved by that the individual tubes 50 are arranged such that, as seen in cross section, their respective centers are the corners of an equilateral triangle. At their contact points or lines 54, two neighboring tubes 50 are attached to each other by medical grade adhesive; alternatively, they might be welded together or otherwise attached to each other by an appropriate method of substance-to-substance bonding.

With respect to exact dimensioning, various alternatives are possible in view of potentially varying requirements. Typically, for catheter systems 1 of the type discussed here, dimensions such as diameters are measured in "French units (Fr)", where one French equals 0.33 mm. Catheter shafts to be used in coronary arteries are preferably between 3 - 5 Fr. in diameter, most preferably around 3 Fr. In contrast, catheter shafts to be used in peripheral vessels are preferably between 5 - 8 Fr. in diameter, most preferably around 5 or 6 Fr. Depending on the exact requirements given by the treatment, for the catheter shaft 2 the dimensions (diameters) are chosen appropriately. In Fig. 20, cross sections of catheter shafts 2 for several typical embodiments are shown.

In the embodiment of Fig. 20a, the outer diameter of the introducer sheath used to introduce the catheter shaft 2 into the blood vessel is assumed to be 6 Fr., i. e. 1.95mm (this size can of course be smaller). In accordance with this criterion, the three-tube shaft design as shown provides a treatment balloon lumen area and an aspiration lumen area of about 0.5 mm² each. In contrast, the occlusion balloons lumen 42, that includes the guidewire lumen 48 as well, has a cross sectional area of about 0.25 mm². In comparison to existing multi-lumen catheter shafts devices, this is a significant increase in flow cross section area or the respective lumen, in consequence allowing for efficient transmission of the respective liquid through the respective cylindrical tube 50.

In the alternative embodiment shown in cross section in Fig. 20b, which discloses a catheter system according to the invention, the ensemble of tubes 50, after the tubes 50 have been bonded with spray medical grade adhesive or alternative substance-to-substance bonding, a thin layer coating 62 (preferably from PTFE) is formed thereon, giving additional strength and protection. Alternatively but not according to the invention, as shown in Fig. 20c, the catheter shaft 2 can be placed inside a separate outer tubing 64 without coating.

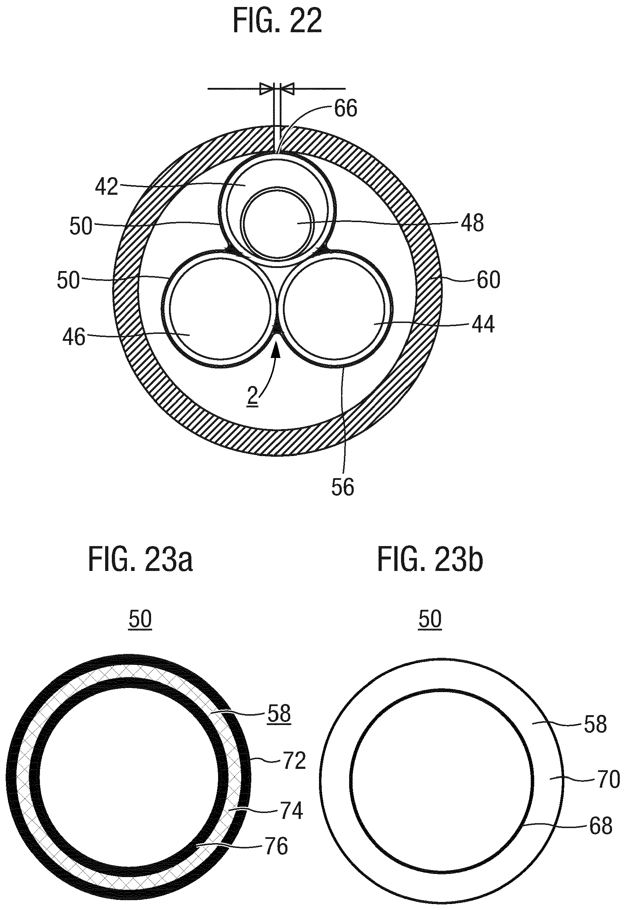

In all cases, sufficient room with respect to the introducer sheath 60 is provided, thereby minimizing friction effects during insertion. Insertion of the multi-lumen catheter shaft 2 into the introducer sheath 60 is shown in cross sectional view in Fig. 21. In the embodiments shown there, reduction of friction between shaft 2 and introducer sheath 60 is already achieved by the very shape of the catheter shaft 2 as such, resulting from the concept of bonding three tubes 50 in the way described, since due to geometry effects, in this design the potential contact area 66 between shaft 2 and introducer sheath 60 is minimized, at the least it is significantly reduced as compared to a conventional circular cross section of the catheter shaft (see Fig. 22: cross sectional view of catheter shaft 2 while being introduced through introducer sheath 60 and touching its inner surface, thereby causing friction). This reduced friction at the surface allows the catheter shaft 2 to move easily in the guiding sheath 60, thereby allowing better and more careful control of the system during introduction.

The tubes 50 forming the catheter shaft 2 in one embodiment are surrounded by an outer coating or wall 56, encapsulating the set of assembled tubes 50.

The individual tubes 50, each providing a single lumen 42, 44, 46, 48, are specifically designed for the specific requirements derived from the intended use in a blood vessel catheter system 1, i. e. regarding rigidity, robustness, sealing properties, biomedical compatibility, etc. In view of these requirements, each tube 50 in the embodiment shown comprises a multi-layered tube wall 58, as shown in the cross sectional view in Fig. 23. In the embodiment shown in Fig. 23b, the tube wall 58 is a two-layer structure, comprising an inner layer 68 made of PTFE and an outer layer 70. The outer layer 70 may be made of PEEK, Polyimide, Nylon 12 material, or the like, and in order to provide good heat shrink laminate performance, yet different material may be chosen.

In another inventive embodiment shown in Fig. 23a, the wall 58 of the respective tube 50 is a multi-layer structure, in which several layers 72, 74, 76 with different functionality are provided. In this embodiment, at least one of said layers, e. g. the middle layer 74, is made from reinforced, e. g. braided, material such as braided PTFE, HDPE, or PFA, thereby providing enhanced mechanical strength, durability, and resistance. In contrast, another of said layers, e. g. the inner layer 76 facing towards the lumen and thereby potentially contacting the medium to be transferred, may be made of material such as PTFE, HDPE, a Polyimide blend, or PFA with low frictional resistance. Yet another layer, e. g. the outer layer 72 surrounding the other layers 74, 76 may be made of materials such as PTFE, Polyimid or PET with low frictional resistance and adding rigidity to the tube system.

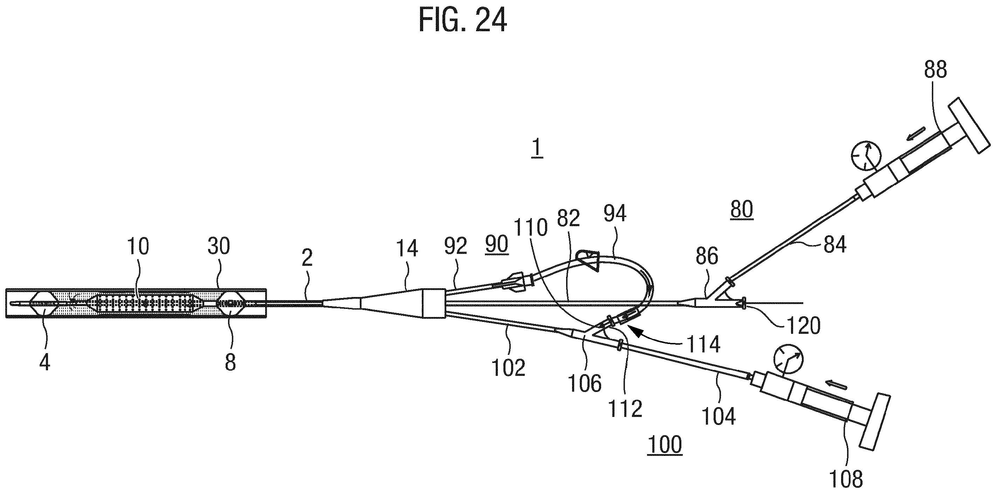

Fig. 24 shows the catheter system 1 when used in recirculation phase, which also (both in setup and regarding the method of use) is considered inventive per se. For such recirculation, the catheter system 1 comprises four main connector lines, each in particular in the embodiment of appropriate tubes or sequences of tubes:
- a first extension line 80 comprising extension tubes 82, 84 connected in sequence to each other via a Y-connector 86, connecting an inflator/deflator device 88, e. g. a pressure controlled in-/deflator pump, or a similar device with the occlusion balloons lumen 42 and therefore with the distal and proximal occlusion balloons 4, 8, to inflate and/or deflate them.
- a second extension line 90 comprising extension tubes 92, 94 connected in sequence to each other, connected with the aspiration lumen 46 and therefore with the aspiration pore 22 between the distal balloon 4 and the perforated balloon 10 in the middle, for aspirating the remaining blood in the isolated area formed by the inflation of the distal and proximal balloon 4, 8. After the drugs and/or liquid were injected into the isolated area by the perforated center balloon 10, it may provide aspiration and/or re-circulation with the respective extension tubes connected to the system.
- a third extension line 100 comprising extension tubes 102, 104 connected in sequence to each other via a Y-connector 106, connecting an inflator/deflator device 108, e. g. a pressure controlled in-/deflator pump, or a similar device, with the treatment balloon lumen 44 and therefore with the perforated treatment balloon 10 to deliver drugs, medication, or other media to the treatment balloon 10. **In** the branch line 110 of the Y connector 106, a one-way valve 112 is provided. In the re-circulation mode shown in Fig. 24, the extension tube 94 with its proximal, free end 114 is connected to the branch line 110 of the Y-connector 106 and thereby to the treatment balloon lumen 44, thereby establishing a closed-loop recirculation system between treatment balloon lumen 44 and aspiration lumen 46. In this mode, the one-way valve 112 prevents the antegrade flow and provides the retrograde flow for re-circulation from aspiration/suction lumen 46 into treatment balloon lumen 44. In this mode, the system is used to transfer drugs and/or fluids from this hub to the isolated area created by the inflation of the distal and proximal balloon and/or provides re-circulation with the extension tubes connected to the system.
- a fourth extension line 120, connected to the Y-connector 86, is connected to the guide wire lumen 48 used to ensure that the balloon catheter 1 reaches the determined area in the vessel 30.

For re-circulation, the catheter shaft 2 is sent to the target area detected in the vessel 30. Then, the distal and proximal balloons 4, 8 are inflated by the inflator or similar device 88 connected to the first extension line 80 connected to the hub. As a consequence of the inflation, the area of the perforated center balloon 10 comprising the lesion 32 in the blood vessel 30 is isolated. Remaining blood in the isolated area can the be aspirated using the second extension line 90. The inflator device 88 or a separate inflation device 108 is then connected to the third extension line 100 in order to inflate the perforated center balloon 10 and/or transfer the drug and/or liquid to the target area. The drug and/or liquid delivered with a controlled pressure begins to further inflate the perforated balloon 10. When the pressure applied reaches a certain level, the drug and/or liquid starts to fill the isolated area formed by the distal and proximal balloons 4, 8 by further inflation through the pores 40 of the perforated balloon 10.

When the filling reaches the appropriate pressure within the second extension line 90 to which it is connected, a retrograde flow occurs within the aspiration channel in between the distal balloon 4 and the perforated center balloon 10. The flow is maintained until it exits the proximal end 114 of the extension tube 94 of the second extension line 90. Afterwards, the proximal end 114 of the extension tube 94 is inserted into the Y-connector 106 connected to the third extension line 100 with the one-way valve 112 that allows a retrograde flow. After this connection, the inflator device 108 connected to the third extension line 100 ensures the recirculation thanks to the pressure created in a controlled manner. Another option may be to connect the second extension line 90 to a circular motor pump. The tube is connected with the rotating part of the motor pump which provides re-circulation by the rotation which is provided, similar to the dialysis device.

### Reference numerals

- 1: Catheter system
- 2: catheter shaft
- 4: distal occlusion balloon
- 6: distal end
- 8: proximal occlusion balloon
- 10: perforated center balloon
- 12: proximal end
- 14: multi-lumen hub
- 16: connector
- 18: extension tubing
- 20: Y-hub
- 22: aspiration pore
- 23,24,25: marker band
- 26: tip
- 30: blood vessel
- 32: lesion
- 34: guidewire
- 40: pores
- 41: arrows
- 42: occlusion balloons lumen
- 44: treatment balloon lumen
- 46: aspiration lumen
- 48: guidewire lumen
- 50,52: tube
- 54: contact point
- 56: wall
- 58: tube wall
- 60: introducer sheath
- 62: coating
- 64: outer tube
- 66: contact area
- 68: inner layer
- 70: outer layer
- 72,74,76: layers
- 80: first extension line
- 82, 84: extension tubes
- 86: Y-connector
- 88: inflator/deflator device
- 90: second extension line
- 92, 94: extension tubes
- 100: third extension line
- 102,104: extension tubes
- 106: Y-connector
- 108: inflator/deflator device
- 110: branch-off line
- 112: one-way valve
- 114: proximal end
- 120: fourth extension line

## Claims

1. Catheter system (1), in particular for use in the treatment and cleaning of arteries, veins, other circulatory channels in the circulatory system of humans and animals, comprising a multi-lumen catheter shaft (2), a distal occlusion balloon (4), a proximal occlusion balloon (8), a central treatment balloon (10) and an aspiration orifice (22) in the distal end region of the catheter shaft (2), wherein an occlusion balloons lumen (42), an aspiration pore lumen (46), and a treatment balloon lumen (44) of the catheter shaft (2) are provided by tubes (50) assembled to each other by adhesive force such that, as seen in cross section, their respective centers are the corners of an equilateral triangle, and wherein the tubes (50) forming the catheter shaft (2) are surrounded by an outer coating (62), encapsulating the set of assembled tubes (50).

2. Catheter system (1) according to claim 1, in which one of the tubes (50) holds a tube (52) for a guidewire (34).

3. Catheter system (1) according to claim 1 or 2, in which the central treatment balloon (10) is a perforated balloon (10).

4. Catheter system (1) according to any one of the claims 1 to 3, in which one or more of the tubes (50) comprises a multi-layered tube wall (58).

## Patentansprüche

1. Kathetersystem (1), insbesondere zur Verwendung bei der Behandlung und Reinigung von Arterien, Venen und anderen Blutgefäßen im Kreislaufsystem von Menschen und Tieren, welches einen mehrlumigen Katheterschaft (2), einen distalen Okklusionsballon (4), einen proximalen Okklusionsballon (8), einen zentralen Behandlungsballon (10) und eine Absaugöffnung (22) in der distalen Endregion des Katheterschaftes (2) umfasst, wobei ein Okklusionsballonlumen (42), ein Absaugporenlumen (46) und ein Behandlungsballonlumen (44) des Katheterschaftes (2) durch Schläuche (50) bereitgestellt werden, die derart durch Adhäsionskraft miteinander verbunden sind, dass im Querschnitt betrachtet ihre jeweiligen Mittelpunkte die Ecken eines gleichseitigen Dreiecks bilden, und wobei die Schläuche (50), welche den Katheterschaft (2) bilden, von einer Außenbeschichtung (62) umgeben sind, welche die Anordnung der verbundenen Schläuche (50) umschließt.

2. Kathetersystem (1) nach Anspruch 1, bei welchem einer der Schläuche (50) einen Schlauch (52) für einen Führungsdraht (34) enthält.

3. Kathetersystem (1) nach Anspruch 1 oder 2, bei welchem der zentrale Behandlungsballon (10) ein perforierter Ballon (10) ist.

4. Kathetersystem (1) nach einem der Ansprüche 1 bis 3, bei welchem einer oder mehrere der Schläuche (50) eine mehrschichtige Schlauchwand (58) umfasst.

## Revendications

1. Système de cathéter (1), en particulier pour l'utilisation dans le traitement et nettoyage des artères, veines et autres voies circulatoires dans le système circulatoire d'humains et d'animaux, comprenant une tige de cathéter multi-lumières (2), un ballonnet d'occlusion distal (4), un ballonnet d'occlusion proximal (8), un ballonnet de traitement central (10) et un orifice d'aspiration (22) dans la région d'extrémité distale de la tige de cathéter (2), dans lequel une lumière (42) des ballonnets d'occlusion, une lumière de pore d'aspiration (46), et une lumière de ballonnet de traitement (44) de la tige de cathéter (2) sont fournies par des tubes (50) assemblés l'un à l'autre par une force adhésive telle que, vu en section transversale, leurs centres respectifs sont les angles d'un triangle équilatéral, et dans lequel les tubes (50) formant la tige de cathéter (2) sont entourés par un revêtement extérieur (62), encapsulant le jeu de tubes assemblés (50).

2. Système de cathéter (1) selon la revendication 1, dans lequel un des tubes (50) maintient un tube (52) pour un fil de guidage (34).

3. Système de cathéter (1) selon la revendication 1 ou 2, dans lequel le ballonnet de traitement central (10) est un ballonnet perforé (10).

4. Système de cathéter (1) selon l'une quelconque des revendications 1 à 3, dans lequel un ou plusieurs des tubes (50) comprend/comprennent une paroi de tube multi-couches (58).
